# EUROPEAN PATENT APPLICATION

(11) **EP 2 444 801 A1**
(43) Date of publication of application: **25.04.2012**
(21) Application number: 10013884.1
(22) Date of filing: 22.10.2010
(51) Int. Cl.: G01N 33/02, G01N 35/00

(54) **Method to control and validate the quality of quantitative analysis of biological samples**

(71) Applicant: Metabolomic Discoveries GmbH, 14476 Potsdam (DE)
(72) Inventor: Schauer, Nicolas, 10117 Berlin (DE)
(74) Representative: Seuss, Thomas

(57) **Abstract**

The described invention relates to a method to control and validate the quality of quantitative chemical analysis of complex sample mixtures comprising various kinds of analytical instruments. Moreover it allows the comparison of different experimental batches over time in a close to matrix environment.

## Description

The described invention relates to a method to control and validate the quality of quantitative chemical analysis of complex sample mixtures comprising various kinds of analytical instruments. Moreover it allows the comparison of different experimental batches over time in a close to matrix environment.

### Background of the invention

Quality control is important to deliver robust results in analytics. Extraction conditions, sample handling and machine sensitivity can influence the results. The variations make sample to sample and experiment to experiment comparisons difficult.

The state of the art is currently to run a defined sample with each batch measurement. This sample can contain a number of compounds, which are naturally also occurring in the sample set. This quality control sample is often made by mixing a number of defined chemicals at a given concentration. This way also calibration curves to quantify defined compounds can be generated.

A common problem in biological sample analysis is the exact determination and quantification of the analyte in question. This is due to a different behavior of the analyte in its natural biological matrix over is synthetically generated matrix as described for the quality control sample. Common problems are shifts in retention time, purity of mass spectra, ion suppression or compound concentrations as well as interaction amongst two ore more compounds to be analyzed.

Recently, quality control samples from biological extracts are being used in identification of compounds in complex samples. These quality control samples are often extracted from a large amount of biological samples e. g. tomato, ketchup, bacteria, yeast or any other type of biological material. This is produced at one time point and then stored. An aliquot is taken with every new sample set. Often this sample has been homogenized and extracted at a defined time point and thus needs to be injected prior, during or post analysis. This so-called bulk quality control sample has the advantage to resemble the same or close biological material being analyzed. Thus the different problems described before can be solved.

Nevertheless this bulk quality control sample has a few drawbacks itself. One of the most important ones is the biological variation of the material. Once it is used up another set has to be prepared. Thus new variation is being introduced and has to be normalized over the experiments between different bulk quality control samples. The storage of bulk quality control samples introduces variation as well, as compounds can be degraded or form by-products. These limitations will not allow doing relative quantifications on the bulk quality control sample or only with labor intensive validation of stability of the sample. Absolute quantification can only be done by using calibration standards with exact concentrations and adding those standard compounds to the bulk quality control sample or biological sample. Here it needs to be validated whether the concentration of the natural compound in the bulk quality control sample does not vary.

### Object of the invention

It is therefore an object of the current invention to improve methods of quantitative chemical analysis, especially of complex sample mixtures such as biological entities. It is an object of the invention to overcome the problems of existing methods such as the biological variation of the bulk material, changes due to storage of the materials and the need for calibration standards.

### Brief description of the invention

The current invention relates to a new quantification process without the problems described above.

It has been found that
a process for parallel quantitative analysis of 10 or more inorganic or organic compounds in a sample, characterized in that,
a matrix comprising
   a natural or synthetic polymer
   a defined amount of the 10 or more inorganic or organic compounds to be analysed,
is quantitatively analysed in addition to said sample and the measured results of the sample and the matrix are compared to each other,
is able to overcome the problems described above.

The process is characterized in that a comparison is made between the actual sample which is to be quantitatively analyzed and a matrix containing 2 or more inorganic or organic compounds in a defined amount.

The matrix may essentially be a natural or synthetic polymer, such as starch, gelatin, pectin, polyethylene, polyethyleneglycole, polyorganosiloxane (silicons), polysaccharides, cellulose, DNA, RNA, polyhydroxalkanoate, polypropylene, polyacrylamide or a mixture thereof.

Said matrix may be in the form of a pill, a capsule, a cube, granulate or any other physical form. The term matrix also includes hollow containers filled with the analyte. The matrix may also include material from a biological entity such as a potato, tomato, blood plasma, urine, microbes and so forth. As explained below under certain circumstances it might be helpful to process two or more different matrices which may be of the same or of different form and/or composition. It might e.g. be helpful to use a gelatin cube (125 mm³) together with a hollow body made of polyethylene containing N-Methyl-N-(trimethylsilyl)trifluoroacetamid.

The matrix contains a defined amount of ten or more inorganic or organic compounds to be analyzed. The number of compounds may be ten or more inorganic or organic compounds. The preferred amount of compounds is 25 or more. It has to be understood that there is no clear upper limit of the number of compounds that are analyzed. In complex situations there may be more than 100 or even several 100 compounds in the matrix. However, it is important that each of the individual compounds contained in the matrix is provided in a defined amount. The compounds may be naturally occurring compounds or they may be chemically synthesized. The compounds may contain stable or radioactive isotopes. The compounds can be in the liquid, gaseous or solid form, and may even change their aggregation state by specific treatment. The compounds may be contained in the natural form or they may be marked with stable or radioactive isotopes. Depending on the number of compounds and their individual physical properties (such as solubility, aggregation form, etc.) the expert skilled in the art is able to select the polymer for the matrix.

It is clear to the expert skilled in the art that the matrix polymer has to be free of the compounds to be measured.

The matrix containing the 10 or more inorganic or organic compounds to be analyzed may be analyzed with a comparable sample of the biological entity. The term biological entity characterizes a sample from a biological product. This may be a plant or part of a plant, such as rice, coffee beans, tomato, wheat, rye, soya or a product from an animal, such as an egg or milk. The sample may also contain animal or human tissue, such as skin, muscle, bone, nerves (incl. spine and/or brain), mucosa, fat, kidney, liver, lung etc.. Said plant, animal or human tissue may be in healthy or pathological form. By way of example: human liver tissue can be in form of healthy liver, in form of liver cirrhosis or in form of cancerous liver. It is furthermore possible to analyze body fluids, such as blood, urine, feces. The biological entity may furthermore be a microbiological entity, such as bacteria, yeast or even a homogenized animal as a whole (such as a fish, a mouse or a rat). The quantitative detection system may be selected from the usual systems for quantitative analysis such as mass spectroscopy, NMR, FID, PDA, fluorescence, or any other detection system. The detection system may be coupled to separation technologies such as GC, LC, CE or any other separation system.

The matrix comprising the defined amount of the 10 or more inorganic or organic compounds to be analyzed may be used to produce calibration curves by using different quantities or by dissolving the matrix in different volumes to generate different concentrations. The expert skilled in the art is aware of the fact, that the calibration curves may be used to confirm the correct selection of matrix material and compounds to be analyzed.

The matrix comprising the defined amount of the 10 or more inorganic or organic compounds to be analyzed may be manufactured according to methods well known in the art. Generally speaking the natural or synthetic polymer or a mixture of several natural or synthetic polymers is contaminated (e.g. contacted, absorbed to or mixed with) with the compounds to be analyzed and divided into small pieces. The compounds to be analyzed may e.g. be solved in an appropriate solvent. The natural or synthetic polymer may be dipped into the solution and the solvent may be removed from the polymer. The resulting matrix comprising the polymer as well as the compounds to be analyzed may then be cut into pieces of (e. g. 100 mg each). It is known to the expert in the art, that it is absolutely essential that a homogeneous distribution of the compounds throughout the matrix material is ensured.

Another way to manufacture the matrices according to the present invention is to carefully warm gelatin to more than 50 °C, to add the compounds to be analyzed under stirring and to cool down the gelatin again. The resulting gelatin block may be cut into pieces as described above. It is known to the expert in the art that the polymer used as the matrix material (such as the natural polymer gelatin described above) is free of compounds that are supposed to be analyzed.

Details of potential manufacturing processes are known from the production of pharmaceutical or dietetic products and do not need to be further explained here.

In special cases two or more matrices may be used in a process according to the present invention. This may be useful in cases where two or more compounds to be analyzed may react with each other. This is e. g. the case when amongst the compounds to be analyzed an ester is present as well as an enzyme which may decompose the ester, such as an esterase. In another example an organic acid could react with a sugar or sugar alcohol present in the sample. In such cases it may be useful to have one matrix comprising one compound (such as the organic acid) and another matrix comprising the other compound (such as the sugar) which will ultimately be analyzed at the same time in the same analytic process by combining both matrices before or during the analytical process. Another example is an incompatibility of a certain compound with the matrix material. In such cases a different matrix material may be used for this compound. A still further example may be a case in which one or more compounds are highly volatile. In such a case it may be necessary to have such volatile compounds in a separate compartment, such as a hollow body made of polyethylene which is filled with the volatile compound(s).

The expert skilled in the art may be faced with other situations in which two or more different matrices may be useful.

Matrices according to the present invention may also be used to convert certain compounds prior to analyses. It is e. g. possible to combine a matrix comprising a compound containing one or more hydroxyl- or amine-groups with a silylating compound (such as trialkylsilylchloride or N-Methyl-N-trimethylsilyltrifluoracetamide) which may be present in a second matrix (optionally combined with an auxiliary base.) Upon dissolution of these matrices the compound containing hydroxyl- or amine-groups may react with the silylating agent so that the hydroxyl- or amine-groups are converted into silyl-groups. This is favorable if the analytical method to be used requires evaporation or stabilization of the compound, e. g. in gas chromatography or liquid chromatography.

One of the main advantages of the process described herein is that the matrix containing the compounds to be analyzed may be used in the same sample preparation method as the actual sample being measured. For example if the quantitative analysis of a number of compounds of a certain vegetable requires an extraction of the compounds prior to quantitative analysis the quality control sample may be processed the same way (i. e. extraction) so that alterations of the quantity of compounds by the extraction process may be calibrated.

The expert skilled in the art is aware of the fact that the concentrations of the various compounds may be very different from each other and may even differ in several magnitudes from each other. A specific compound may occur in a given biological entity as several grams per kilogram whereas another compound may occur only in several nanograms per kilogram. It is therefore recommendable to select the defined amount of a compound to be analyzed in the matrix in the same magnitude as it is expected in the analyte.

The present invention is further illustrated in the examples below.

### Examples

### Example 1 - Aroma/Volatile Analysis in Coffee Beans

The analysis of coffee beans on volatile composition using analytical instruments is a time consuming and complex task. Repeated analysis of the same or different samples over a day or after several days is common. A general problem in quantification and normalization of sample data taken over several days exists. Problems arise through variation in sample preparation and in technical variation caused by the analytical equipment employed. A solution is to use internal or external standards, which can be purchased as pure, isolated compounds. This procedure is time consuming, should contain a large number of compounds interested in and lacks the typical sample matrix. The missing sample matrix is leading to false representation of concentrations or levels of compounds, because interaction between the polymeric matrix and several compounds is not possible. Here we describe a novel approach to overcome this hurdle.

A sample matrix can consist of organic or synthetic polymers. In this example pectin is used to resemble this sample matrix. The matrix is formed as a gel capsule and encloses for instance a set of 19 aroma compounds typically present in coffee beans with a defined concentration (Table 1).

**Table 1: List of coffee bean volatiles**

| | | |
|---|---|---|
| E)-ß-Damascenone | E)-ß-Damascenone | 3-Hydroxy-4,5-dimethyl- 2(5H)-furanone (Sotolon) |
| 2-Furfurylthiol | 2-Furfurylthiol | 4-Ethylguaiacol |
| 3-Mercapto-3-methylbutylformate | 4-Vinylguaiacol | 5-Ethyl-3-hydroxy-4-methyl-2(5H)-furanone (Abhexon) |
| 3-Methyl-2-buten-1-thiol | 2,3-Pentanedione | 2-Ethyl-3,5-dimethylpyrazine |
| 2-Isobutyl-3-methoxypyrazine | Methional | 2,3-Diethyl-5-methylpyrazine |
| 5-Ethyl-4-hydroxy-2-methyl-3(2H)-furanone | 2-Isopropyl-3-methoxypyrazine | 4-Hydroxy-2,5-dimethyl- 3(2H)-furanone (Furaneol) |
| Guaiacol | Vanillin | 2,3-Butanedione (diacetyl) |

This so called quality control sample allows the same treatment procedure as every coffee bean sample for volatile analysis. For instance, when the volatiles from the coffee bean are being extracted using heat and a solvent, like water, the same treatment can occur to the quality control sample. This allows the same treatment as compared to biological samples and making comparison of experimental and technical variation possible.

### Example 2 - Compound Analysis in Rice

Rice contains several hundred up to thousands of chemical compounds. For the analysis of those compounds analytical instruments are being used. Often only a few, but sometimes a multiple of those compounds shall be investigated at the same time. Common problems in day to day analysis are variations in sample preparation and equipment performance. The comparison of samples run on different days or even on different instruments is nearly impossible with existing methods known in the art. One way to solve this is to run a standardized rice sample along with each analysis. As each biological sample as its own variation a large batch of this standardized sample needs to be prepared and stored. Upon storage degradation, oxidation and other chemical reactions can occur and thus making this sample not as robust as needed. Another way could be the preparation of a chemical standard mix with several folds of the rice containing compounds. This is very time-consuming and expensive and at the same time does not resemble the rice sample, with all its matrix effects, like reactions with the polymeric compounds in the sample, desorption, side reactions, ion suppression and so on.

A solution to this problem is to produce a standard sample in the form of granulate, which is made out of a rice polymer, for instance starch in which tens to hundreds of compounds with a defined concentration are embedded. A list of such a standard sample is shown in table 2. This standard sample can be prepared alongside the rice samples under the exact same conditions and thus allows unique comparison individual samples over several experiments. This sample can also serve purpose to establish a calibration curve for the absolute quantification of compounds.

**Table 2: List of common compounds in rice**

| Adenosine | Citric acid | Glucose | Glycine |
|---|---|---|---|
| Inositol-2-phosphate, myo- | Nonanoic acid | Ornithine-1,5-lactam | Alanine |
| Erythritol | Glucose, 2-amino-2-deoxy-, D- | Gulonic acid, 2-oxo-, DL- | Isoleucine |
| Homoserine | Pentacosane, n- | Alanine, beta- | Fructose |
| Glucuronic acid-3,6-lactone | Heptacosane, n- | Lactic acid | Octadecadienoic acid, 9,12-(Z,Z)- |
| Phosphoric acid | Arabinose | Fumaric acid | Glutamic acid |
| Succinic acid | Laminaribiose | Octadecadienoic acid, n- | Proline |
| Aspartic acid | Galactitol | Glutamine | Hexadecanoic acid |
| Malic acid | Octadecanoic acid | Putrescine | Cadaverine |
| Galactose | Tricosane, n- | Hexadecenoic acid, 9-(Z)- | Maltose |
| Octadecatrienoic acid, 6,9,12-(Z,Z,Z)-, n- | Pyridine, 2-hydroxy- | Lysine | Glucaric acid-1,4-lactone |
| Glycerol | Homoserine lactone | Mannitol | Octadecenoic acid, 9-(Z)- |
| Pyroglutamic acid | Diethylenglycol | Glucopyranose, D- | Glycerol-3-phosphate |
| Inositol, myo- | myo-Inositol-1-phosphate | Ornithine | Pyruvate |
| Glutaric acid, 2-oxo- | Asparagine | Aconitic acid, cis- | Tyrosine |
| Tryptophan | Phenylalanine | Isocitric acid | Serine |
| Shikimic acid | Sorbitol | Leucine | Threonine |
| Trehalose, alpha,alpha'-, D- | Butyric acid, 4-amino- | Valine | Uracil |

### Example 3 - Compound analysis in rice using a natural matrix

The described compound analysis in rice in example 2 can also be performed using rice as a matrix and adding typical rice standard compounds in a labeled form, this can be either using stable isotopes, like ¹³C, ²H (Deuterium) or ¹⁵N or radioactive labeled molecules, like ¹⁴C. This sample can then be provided as granulate, a pill, a cube or any other form.

### Example 4 - Compound analysis in rice using a stable isotope labeled standard mixture

Given rice example can be standardized using a rice quality batch with a defined quantity of compounds. This quality batch can be grown under controlled ¹³C atmosphere (containing CO₂ marked with ¹³C, i.e. ¹³CO₂) allowing full labeling of carbon containing compounds in rice with ¹³C. This is different to normal atmospheric conditions where a ratio of ¹²C/¹³C is incorporated into organic compounds in planta. The described batch is then extracted and defined quantities of this extract will then be aliquoted into different sizes and provided in the form of granulate, a pill, a cube or any other form in a defined matrix. The composed quality control standard can then be analyzed alongside samples or added to the samples of interest.

Similar results can be achieved with plants grown in an environment containing isotopes from various elements (e.g. H, C, N, O, P, S) in form of isotope labeled compounds such as ¹⁵NH₃, ¹⁵NH₄Cl, (¹⁵NH₄)₂CO₃, D₂O, H₂¹⁸O, K₃³²PO₄, H₂³⁴S, ¹⁸O₂, ³⁴SO₂, C¹⁸O₂ or ¹⁴CO₂. The isotope may be stable or unstable (radioactive). The expert skilled in the art may select the isotope label according to the analytical task and selected method. The expert skilled in the art will moreover appreciate that the environment does not have to contain 100% of the isotope labeled compound, it may be sufficient to have a higher content of the isotope labeled compound in the environment compared to standard (natural) conditions.

## Claims

1. A process for parallel quantitative analysis of 10 or more inorganic or organic compounds in a sample, **characterized in that**,
a matrix comprising
a natural or synthetic polymer
a defined amount of the 10 or more inorganic or organic compounds to be analysed,
is quantitatively analysed in addition to said sample and the measured results of the sample and the matrix are compared to each other.

2. A process according to claim 1, **characterized in that** the matrix essentially consists of gelatin, polyethylene, polyethyleneglycole, polyorganosiloxane (silcons), polysaccharides, cellulose, DNA, RNA, polyhydroxalkanoate, polypropylene, polyacrylamide, starch, pectine or any other polysaccharide or mixtures of these polymers.

3. A process according to claim 1 **characterized in that** the matrix comprises 25 or more inorganic or organic compounds.

4. A process according to claim 1 **characterized in that** the matrix comprises 50 or more inorganic or organic compounds.

5. A process according to claim 1, **characterized in that**
the sample is gained from a biologic entity and the matrix comprises 10 or more inorganic or organic compounds usually expected in a biological entity.

6. A process according to claim 5, **characterized in that**
the matrix comprises at least one isotope labeled inorganic or organic compound.

7. A process according to claim 6, **characterized in that**
the isotope labeled inorganic or organic compound is labeled with an isotope of an element selected from H, C, P, N, 0, S.

8. A process according to claim 5, **characterized in that** the biological entity is rice, coffee beans, tomato, wheat, rye, soya, egg, animal tissue, bacteria, funghi, algae, animal or human tissue, such as skin tissue, muscle tissue, bone tissue, nerve tissue (incl. spine and/or brain), mucosa tissue, fat tissue, kidney tissue, liver tissue, lung tissue.

9. A process according to claim 8 **characterized in that** the tissue is in healthy or pathological form.

10. A process according to claim 8 **characterized in that** the matrix contains at least one one isotope labeled inorganic or organic compound which is gained from a biological entity grown under standardized conditions in an isotope labeled environment.

11. A process according to claim 5 **characterized in that** the matrix comprises 10 or more inorganic or organic compounds in an amount which is similar to the expected amount usually found in the biological entity.

12. A matrix for the use in a process according to claim 1,
comprising
a natural or synthetic polymer
a defined amount of the 10 or more inorganic or organic compounds to be analysed.

13. A matrix according to claim 12, **characterized in that** the matrix essentially consists of gelatin, polyethylene, polyethyleneglycole, polyorganosiloxane (silcons), polysaccharides, cellulose, DNA, RNA, polyhydroxalkanoate, polypropylene, polyacrylamide, starch, pectine or any other polysaccharide or mixtures of these polymers.

14. A matrix according to claim 12, **characterized in that** the matrix comprises 25 or more inorganic or organic compounds, preferably 100 or more inorganic or organic compounds.

15. A matrix according to claim 12 **characterized in that** the matrix comprises at least one isotope labeled inorganic or organic compound.
